# EUROPEAN PATENT SPECIFICATION

(11) **EP 2 451 459 B1**
(45) Date of publication and mention of the grant of the patent: **22.11.2017**
(21) Application number: 10797804.1
(22) Date of filing: 07.07.2010
(51) Int. Cl.: A61K 31/337, A61K 31/6615, A61K 33/24, A61K 31/282, A61K 31/4745, A61K 31/513, A61K 31/7068, A61P 35/00

(54) **METHOD OF REDUCING MULTI-DRUG RESISTANCE USING INOSITOL TRIPYROPHOSPHATE**
VERFAHREN ZUR REDUZIERUNG EINER MULTIRESISTENZ GEGEN ARZNEIMITTEL MITTELS INOSITOL-TRIPYROPHOSPHAT
MÉTHODE DE RÉDUCTION D'UNE RÉSISTANCE MULTIMÉDICAMENTEUSE À L'AIDE D'INOSITOL TRIPYROPHOSPHATE

(30) Priority: 07.07.2009 US 223583 P
(43) Date of publication of application: 16.05.2012
(73) Proprietor: Normoxys, Inc., Wellesley, MA 02481 (US); Université de Strasbourg, 67000 Strasbourg (FR)
(72) Inventor: NICOLAU, Yves, Claude, Newton MA 02459 (US); LEHN, Jean-marie, F-67000 Strasbourg (FR); KIEDA, Claudine, F-45000 Orleans (FR)
(74) Representative: Grund, Martin
(86) International application number: PCT/US2010/041250
(87) International publication number: WO 2011/005886

(56) References cited:
- WO-A1-2006/102060
- WO-A1-2007/081315
- FYLAKTAKIDOU K C ET AL: "Inositol tripyrophosphate: a new membrane permeant allosteric effector of haemoglobin", BIOORGANIC & MEDICINAL CHEMISTRY LETTERS, PERGAMON, GB, vol. 15, no. 6, 15 March 2005 (2005-03-15) , pages 1605-1608, XP004771154, ISSN: 0960-894X, DOI: 10.1016/J.BMCL.2005.01.064
- SAIARDI ADOLFO ET AL: "Inositol pyrophosphates regulate cell death and telomere length through phosphoinositide 3-kinase-related protein kinases", PROCEEDINGS OF THE NATIONAL ACADEMY OF SCIENCES OF THE UNITED STATES OF AMERICA, vol. 102, no. 6, 8 February 2005 (2005-02-08), pages 1911-1914, XP002715367, ISSN: 0027-8424
- NG S S W ET AL: "INHIBITION OF PHOSPHATIDYLINOSITIDE 3-KINASE ENHANCES GEMCITABINE-INDUCED APOPTOSIS IN HUMAN PANCREATIC CANCER CELLS", CANCER RESEARCH, AMERICAN ASSOCIATION FOR CANCER RESEARCH, US, vol. 60, no. 19, 1 October 2000 (2000-10-01), pages 5451-5455, XP001084246, ISSN: 0008-5472
- HUANG CHUANSHU ET AL: "Inositol hexaphosphate inhibits cell transformation and activator protein 1 activation by targeting phosphatidylinositol-3' kinase", CANCER RESEARCH, vol. 57, no. 14, 1997, pages 2873-2878, XP002715368, ISSN: 0008-5472
- BIOLO, A. ET AL.: 'Enhanced exercise capacity in mice with severe heart failure treated with an allosteric effector of hemoglobin, myo-inositol trispyrophosphate' PROCEEDINGS OF THE NATIONAL ACADEMY OF SCIENCES OF THE UNITED STATES OF AMERICA vol. 106, no. 6, 2009, pages 1926 - 1929, XP008151127
- KIEDA, C. ET AL.: 'Suppression of hypoxia-induced HIF-1alpha and of angiogenesis in endothelial cells by myo-inositol trispyrophosphate-treated erythrocytes' PROCEEDINGS OF THE NATIONAL ACADEMY OF SCIENCES OF THE UNITED STATES OF AMERICA vol. 103, no. 42, 2006, pages 15576 - 15581, XP008121655

## Description

### RELATED APPLICATION

This application claims the benefit of U.S. Provisional Application No. 61/223,583, filed July 7, 2009.

### BACKGROUND

Cancer is one of the leading causes of death in the developed world, resulting in over 500,000 deaths per year in the United States alone. Over one million people are diagnosed with cancer in the U.S. each year, and overall it is estimated that more than 1 in 3 people will develop some form of cancer during their lifetime. Solid tumors account for more than 85% of cancer mortality.

Angiogenesis have been associated with a number of different types of cancers. Angiogenesis is controlled through a highly regulated system of angiogenic stimulators and inhibitors. The control of angiogenesis is altered in certain disease states and, in many cases, pathological damage associated with the diseases is related to uncontrolled angiogenesis. Both controlled and uncontrolled angiogenesis are thought to proceed in a similar manner. Endothelial cells and pericytes, surrounded by a basement membrane, form capillary blood vessels. Angiogenesis begins with the erosion of the basement membrane by enzymes released by endothelial cells and leukocytes. Endothelial cells, lining the lumen of blood vessels, then protrude through the basement membrane.

Angiogenic stimulants induce the endothelial cells to migrate through the eroded basement membrane. The migrating cells form a "sprout" off the parent blood vessel where the endothelial cells undergo mitosis and proliferate. The endothelial sprouts merge with each other to form capillary loops, creating a new blood vessel.

Persistent, unregulated angiogenesis occurs in many disease states, tumor metastases, and abnormal growth by endothelial cells. The diverse pathological disease states in which unregulated angiogenesis is present have been grouped together as angiogenic-dependent or angiogenic-associated diseases.

The hypothesis that tumor growth is angiogenesis-dependent was first proposed in 1971. In its simplest terms, this hypothesis states: "Once tumor "take" has occurred, every increase in tumor cell population must be preceded by an increase in new capillaries converging on the tumor." Tumor "take" is currently understood to indicate a prevascular phase of tumor growth in which a population of tumor cells occupying a few cubic millimeters volume, and not exceeding a few million cells, can survive on existing host microvessels. Expansion of tumor volume beyond this phase requires the induction of new capillary blood vessels. For example, pulmonary micrometastases in the early prevascular phase in mice would be undetectable except by high power microscopy on histological sections.

Angiogenesis has been associated with a number of different types of cancer, including solid tumors and blood-borne tumors. Solid tumors with which angiogenesis has been associated include, but are not limited to, rhabdomyosarcomas, retinoblastoma, Ewing's sarcoma, neuroblastoma, and osteosarcoma. Angiogenesis is also associated with blood-borne tumors, such as leukemias, any of various acute or chronic neoplastic diseases of the bone marrow in which unrestrained proliferation of white blood cells occurs, usually accompanied by anemia, impaired blood clotting, and enlargement of the lymph nodes, liver and spleen. It is believed that angiogenesis plays a role in the abnormalities in the bone marrow that give rise to leukemia tumors and multiple myeloma diseases.

As mentioned above, several lines of evidence indicate that angiogenesis is essential for the growth and persistence of solid tumors and their metastases. Once angiogenesis is stimulated, tumors upregulate the production of a variety of angiogenic factors, including fibroblast growth factors (aFGF and bFGF) and vascular endothelial growth factor/vascular permeability factor (VEGF/VPF).

The role of VEGF in the regulation of angiogenesis has been the object of intense investigation. Whereas VEGF represents a critical, rate-limiting step in physiological angiogenesis, it appears to be also important in pathological angiogenesis, such as that associated with tumor growth. VEGF is also known as vascular permeability factor, based on its ability to induce vascular leakage. Several solid tumors produce ample amounts of VEGF, which stimulates proliferation and migration of endothelial cells, thereby inducing neovascularization. VEGF expression has been shown to significantly affect the prognosis of different kinds of human cancer. Oxygen tension in the tumor has a key role in regulating the expression of VEGF gene. VEGF mRNA expression is induced by exposure to low oxygen tension under a variety of pathophysiological circumstances.

Growing tumors are characterized by hypoxia, which induces expression of VEGF and may also be a predictive factor for the occurrence of metastatic disease. It is also recognized that, unlike normal blood vessels, tumor vasculature has abnormal organization, structure, and function. Tumor vessels are also found to be leaky and blood flow is heterogeneous and often compromised.

Because cancer cells require access to blood vessels for growth and metastasis, it is believed that inhibiting angiogenesis offers hope for treating cancers and tumors. However, the anti-angiogenic strategies have been explored to date, without providing lasting therapeutic benefits, because of the resulting selection of drug resistant, highly aggressive metastatic cancer cells. Such anti-angiogenic treatments that destroy tumor vascularisation are found, in some cases, to enhance metastatic invasion.

What is needed, therefore, is a substantially non-toxic composition and method that can regulate tumor blood vasculature. Also, an improved cancer therapy is needed.

### SUMMARY

The invention is defined by the appended claims. The data provided in the Examples indicate that blood vessel normalization in combination with chemotherapy is a potentially beneficial approach to cancer therapy. Inositol trisphosphate (ITPP), an allosteric effector of haemoglobin, enhances oxygen release, counteracts the effects of hypoxia and inhibits angiogenesis in vitro. In a mouse model, ITPP in red blood cells (ITPP-RBCs) reduces lung metastasis induced by intravenous injection of mouse melanoma cells. ITPP, associated with the chemotherapeutic agents cisplatin and paclitaxel, inhibited primary melanoma growth and lung metastases. In a rat model of pancreatic adenocarcinoma, ITPP used in conjunction with gemcitabine caused a significant rise in the survival rate of tested animals, showing a strong additive effect. ITPP also significantly enhances the infiltration of macrophages and natural killer cells into tumors.

Disclosed is a method for treating cancer, comprising administering to a subject in need thereof a therapeutically effective amount of ITPP; and administering to the subject a therapeutically effective amount of a chemotherapeutic agent following the partial vascular normalization in the tumor.

In one aspect, disclosed is a pharmaceutical composition comprising inositol trispyrophosphate (ITPP) and a chemotherapeutic agent, such as those selected from paclitaxel, cisplatin and gemcitabine.

In another aspect, disclosed is a treatment regimen for treating cancer in a subject, comprising administering simultaneously or sequentially a therapeutically effective amount of ITPP and a chemotherapeutic agent, such as those selected from paclitaxel, cisplatin and gemcitabine.

In another aspect, disclosed is a pharmaceutical composition comprising ITPP and a subtherapeutic amount of a chemotherapeutic agent.

In yet another aspect, disclosed is a treatment regimen or a method for treating cancer in a subject, comprising administering simultaneously or sequentially a therapeutically effective amount of ITPP and a sub-therapeutic amount of a chemotherapeutic agent.

In a further aspect, disclosed is a method of treating a cancer that is resistant to one or more chemotherapeutic agents by administering a therapeutically effective amount of ITPP. In certain embodiments, the cancer is resistant to paclitaxel and/or cisplatin.

Disclosed is also a method for treating a hyper-proliferative condition comprising administering to a subject in need thereof a therapeutically effective amount of ITPP, wherein the hyper-proliferative condition is not cancer or characterized by undesired angiogenesis.

Further disclosed is a method for enhancing immune response in a subject, comprising administering to a subject in need thereof a therapeutically effective amount of ITPP, wherein the subject does not suffer from cancer or another tumor.

In addition, disclosed is the use of the compositions described herein in medicine and the use of the compositions described herein in the manufacture of a medicament for treating a condition described herein.

### BRIEF DESCRIPTION OF THE DRAWINGS

Figures not pertaining to the invention are for illustrative purposes only.

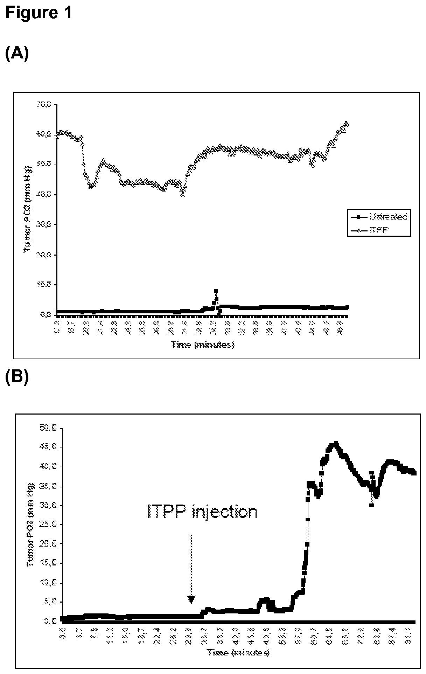

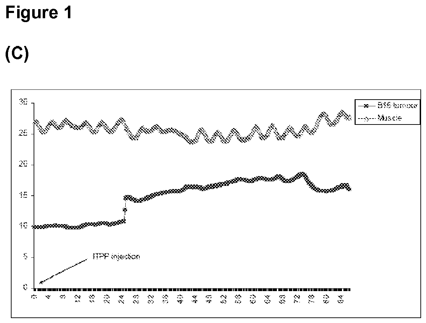

**FIG. 1** shows ITPP-red blood cell (RBC)-induced selective increase of oxygen pressure in a developed subcutaneous melanoma tumor. (A) Comparison between untreated tumor implanted 14 days before and the same tumor treated with ITPP at day 12 and 13. Note the pO₂ level 24 hours after the ITPP injection. (B) Time lapse recording of pO₂ in subcutaneously implanted tumor before and after intra peritoneal injection of ITPP. Note the oxygen pressure increase 30 min after treatment. (C) ITPP does not affect muscle pO₂. Intra peritoneally injected ITPP increases pO₂ in hypoxic subcutaneous tumor (lower curve) but does not affect the pO2 in healthy muscle (upper curve). Data from one representative experiment out often conducted with ten mice per group.

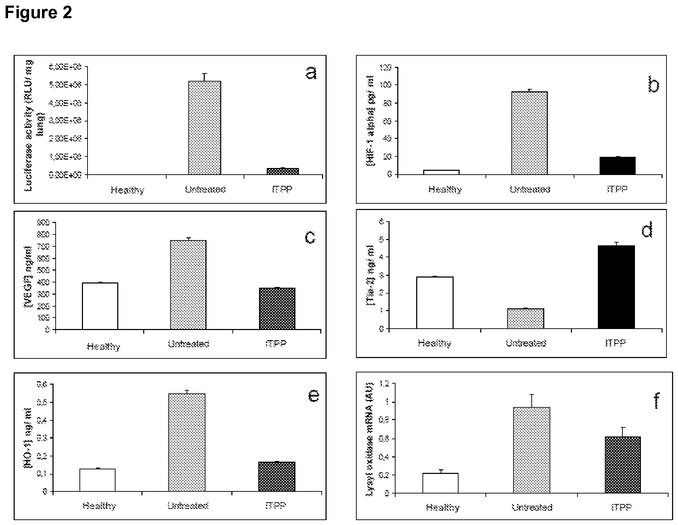

**FIG. 2** shows oxygen supply by ITPP-RBC inhibits lung metastasis and reverses hypoxia-induced genes cascade in experimental melanoma model. Protein and enzymatic activity measurements, in lung lysates from melanoma bearing untreated (grey) and ITPP-treated (black) mice compared to healthy controls (white), on day 27 after melanoma inoculation : (A) Lung metastases quantification by Luciferase assay. (B) HIF- 1α expression; (C) VEGF expression. (D) Tie-2 and (E) HO-1 expression, estimation by ELISA on day 19 after melanoma cells injection. (F) mRNA LOX content, estimation. Data are mean values calculated from 8 to 10 separate mice per group from one representative experiment out of 5.

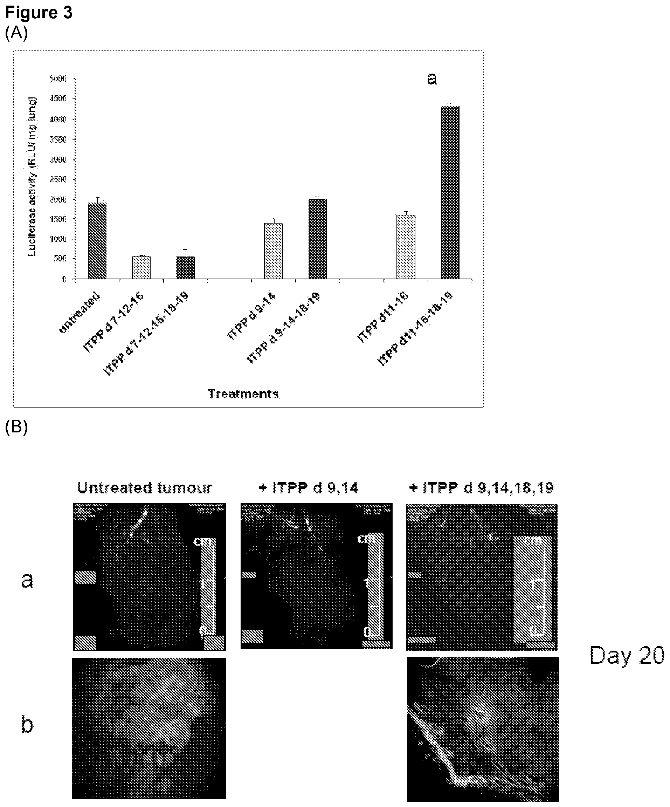

**FIG. 3** shows that the ITPP treatment schedule can affect anti metastatic activity, vessel normalisation and reduction of multi drug resistant cell level in mice with subcutaneous melanoma. (A) Effect of starting time and duration of ITPP treatment before drug application at days 20, 21. ITPP reduces metastases if started at day 7 and is less efficient later with an increase in metastases upon chronic treatment. The luciferase analysis was at day 25. Data are mean values calculated from ten separate mice per group from one representative experiment out of 10. (B) Effect of ITPP treatment on tumor vessel normalization assessed at day 20, by: (a) Magnetic Resonance Imaging of vessels architecture in subcutaneous, untreated tumors compared to ITPP treated mice. Note the well organized vasculature (arrows) after ITPP treatment (day 9,14,18,19) compared to the disorganized discontinuous vessels (arrows) in the non treated tumors, (b) Immunostaining by anti-SMA antibodies of pericytes around the normalized vessels compared to control.

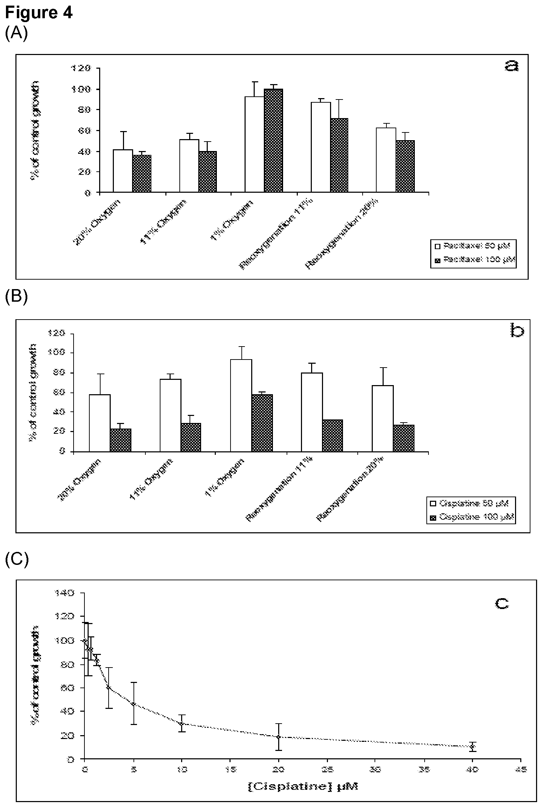

**FIG. 4** shows chemosensitivity, in vitro, of mouse melanoma cells upon hypoxia and reoxygenation and mouse of lung endothelial. The sensitivity of melanoma cells to chemotherapeutic drugs: (A) Paclitaxel; (B) cisplatin, is abolished by hypoxia. This is reverted upon reoxygenation of the cells. (C) Endothelial cell sensitivity assessment toward cisplatin.

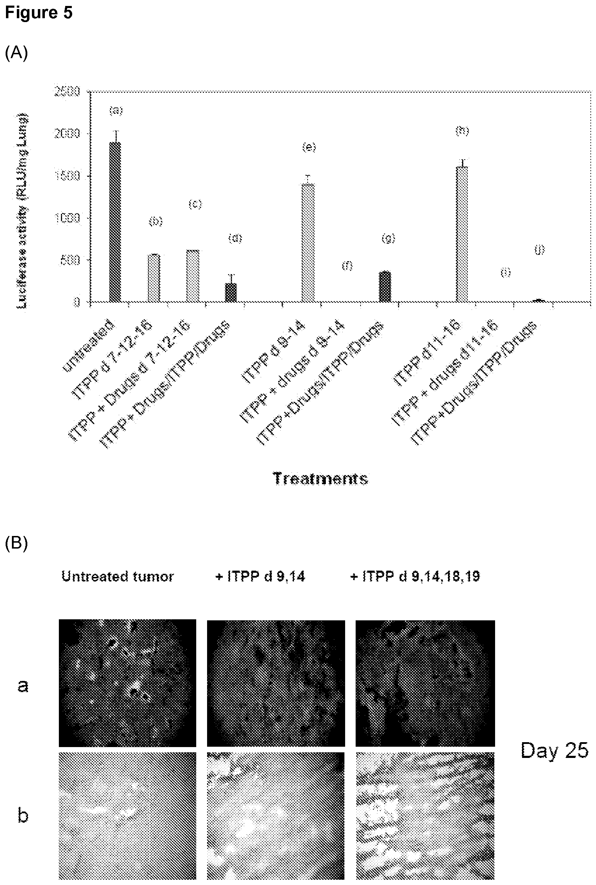

**FIG. 5** shows tumor oxygenation and vessel normalization improve chemotherapy in melanoma. (A) ITPP, paclitaxel and cisplatin combination reduces metastasis according to the chronology of the treatments. Eradication of lung metastases was obtained upon reinjection of ITPP (days 18, 19) before drug reinjections (days 20 and 21). (a) = untreated; (b) = ITPP days 7, 12, 16; (c) = « b » + drugs days 7, 12, 16; (d) = « c » + ITPP days 18,19 + drugs days 20,21; (e) = ITPP days 9, 14; (f) = « e » + drugs days 9,14; (g) = « f » + ITPP days 18, 19 +drugs days 20, 21; (h) = ITPP 11, 16; (i) = « h » + drugs days 11, 16; (j) = « i » + ITPP days 18, 19 + drugs days 20, 21. The luciferase activity was analysed at day 25. Data are mean values calculated from ten separate mice per group from one representative experiment out of 10. (B) Metronomic combination effect of ITPP-induced normalization on drug chemotherapeutic activity. Two groups of mice were treated by ITPP either at days 9, 14 or days 9, 14, 18, 19. The two groups of ITPP-treated mice received paclitaxel and cisplatin at day 20, 21. Tumors were stained at day 25. (a) Immunostaining of vessels by CD31 (al) in non-treated tumors compared to ITPP and drug-treated mice in a2 and a3 CD31+ staining corresponds to necrotic areas, (b) Hematoxylin-eosin staining of tumors of mice treated as in (a). Note the efficient necrotic destruction of the tumor upon treatment (a3, b3). Data are representative for experiments performed on 10 mice per group.

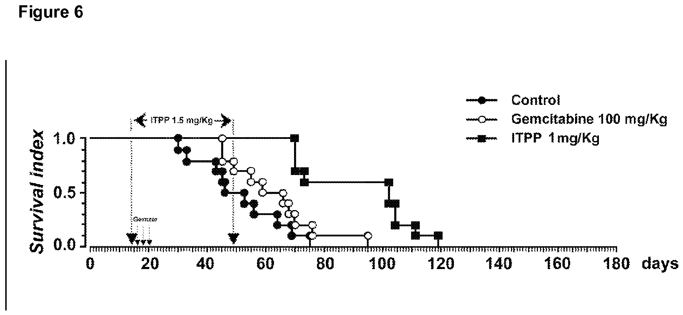

**FIG. 6** shows the effect of ITPP treatment on survival of rats with pancreatic tumor, as compared to the effect of gemcitabine treatment alone and placebo. In the ITPP treatment group, rats with pancreatic tumor were treated with ITPP (1.5 mg/Kg) weekly during the period of day 14 to day 49. In the gemcitabine treatment group, rats with pancreatic tumor were treated with gemcitabine (100 mg/Kg) on days 16, 18 and 20. Animals in the control group were not treated.

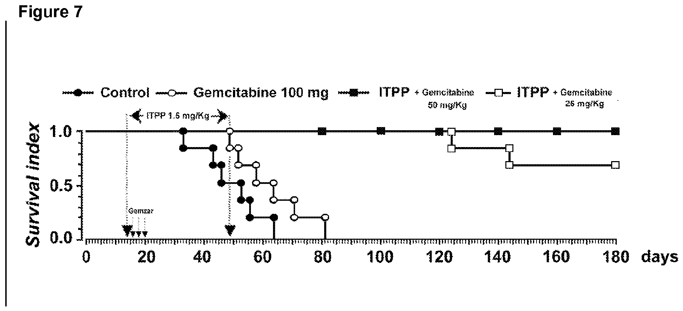

**FIG. 7** shows the effect on survival of rats with pancreatic tumor using hexasodium myo-inositol trispyrophosphate (OXY111A) in combination with gemcitabine, as compared to the effect of gemcitabine treatment alone and placebo. In the combination treatment group, rats with pancreatic tumor were treated with ITPP (1.5 mg/Kg) in combination with gemcitabine (25 mg/Kg or 50 mg/Kg) weekly during the period of day 14 to day 49. In the gemcitabine treatment group, rats with pancreatic tumor were treated with gemcitabine (100 mg/Kg) on days 16, 18 and 20. Animals in the control group were not treated.

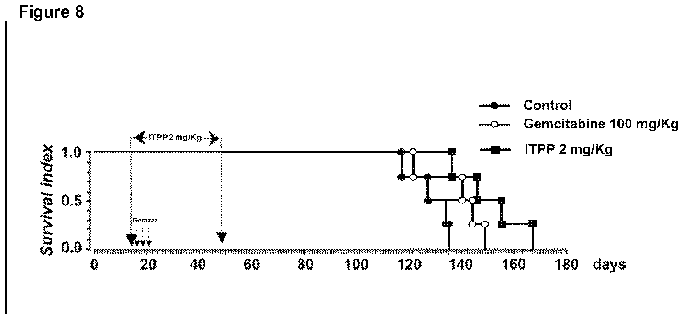

**FIG. 8** shows the effect of ITPP treatment on survival of nude mice with Human Panc-1 pancreatic tumor xenograft, as compared to the effect of gemcitabine treatment alone and placebo. In the ITPP treatment group, mice with tumor xenograft were treated with ITPP (2 mg/Kg) weekly during the period of day 14 to day 49. In the gemcitabine treatment group, mice with tumor xenograft were treated with gemcitabine (100 mg/Kg) on days 16, 18 and 20. Animals in the control group were not treated.

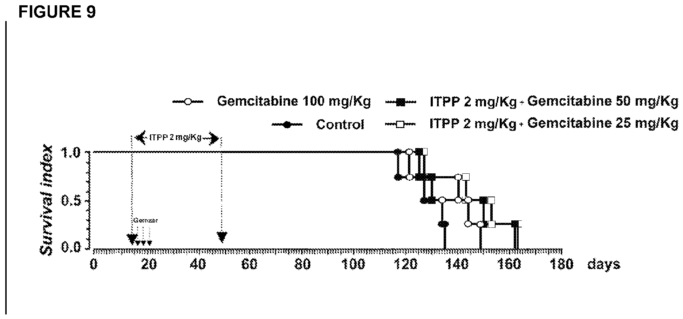

**FIG. 9** shows the effect on survival of nude mice with Human Panc-1 pancreatic tumor xenograft using ITPP in combination with gemcitabine, as compared to the effect of gemcitabine treatment alone and placebo. In the combination treatment group, mice with tumor xenograft were treated with ITPP (2 mg/Kg) in combination with gemcitabine (25 mg/Kg or 50 mg/Kg) weekly during the period of day 14 to day 49. In the
   gemcitabine treatment group, mice with tumor xenograft were treated with gemcitabine (100 mg/Kg) on days 16, 18 and 20. Animals in the control group were not treated.

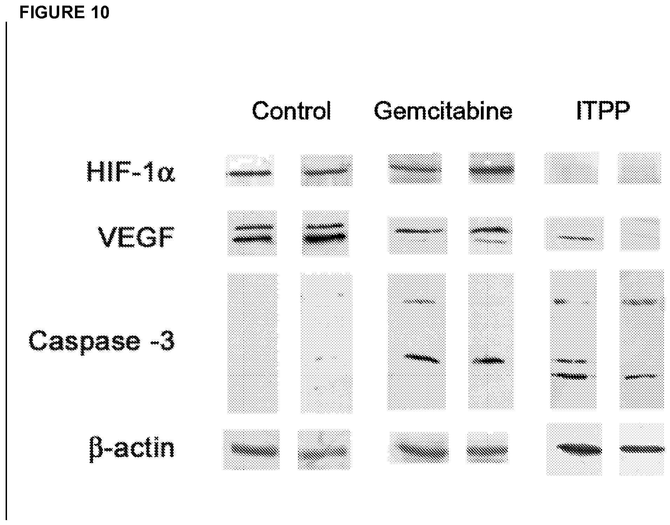

**FIG. 10** shows the effect of ITPP treatment on expression of HIF- 1α, VEGF, caspase-3 and β-actin in rats with pacreatic tumor, as compared to the effect of gemcitabine treatment alone and placebo.

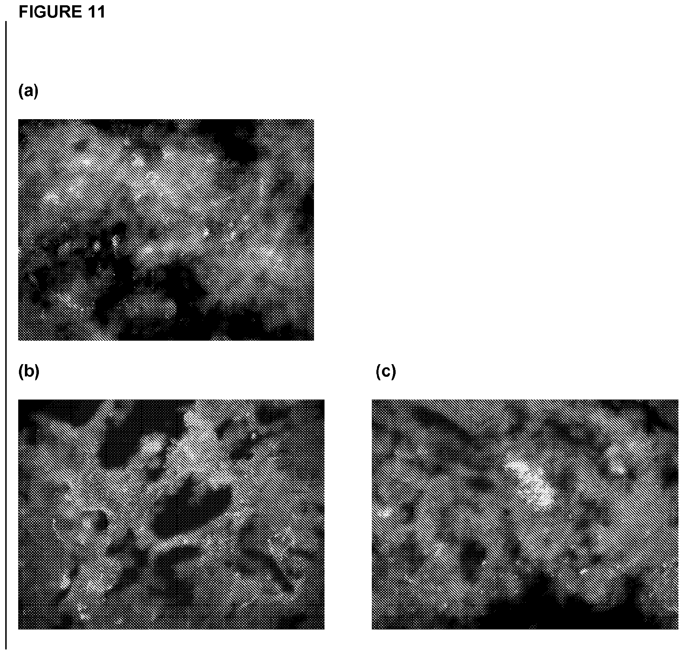

**FIG. 11** shows the effect of ITPP treatment on infiltration of the CD68 (M2 type) macrophage into the B16 tumor. OXY111A was injected intraperitoneally on days 7,8, 14, 15, 21,22, 29 and 30. Analysis of the B16 tumor was performed on day 31. (a) untreated B16 tumor; (b) and (c) CD68 staining of ITPP treated tumor shows CD68 (M2 type) macrophage infiltration into the B16 tumor.

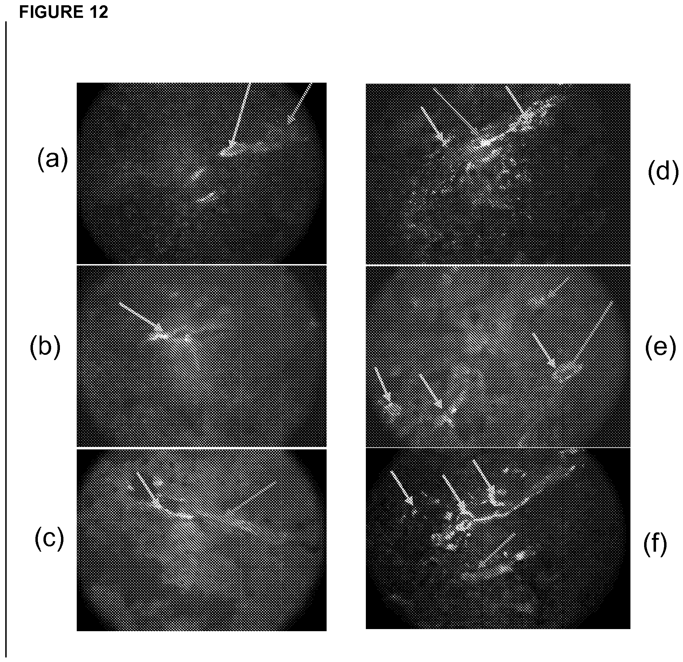

**FIG. 12** shows the effect of ITPP treatment on infiltration of the CD49b natural killer (NK) cells and on the presence of CD31 endothelial (EC) cells in the B16 tumor. (a) to (c) untreated B16 tumor; (d) to (f) B16 tumor treated with ITPP. Green arrows indicate the infiltrating NK cells; red arrows indicate the vessel walls.

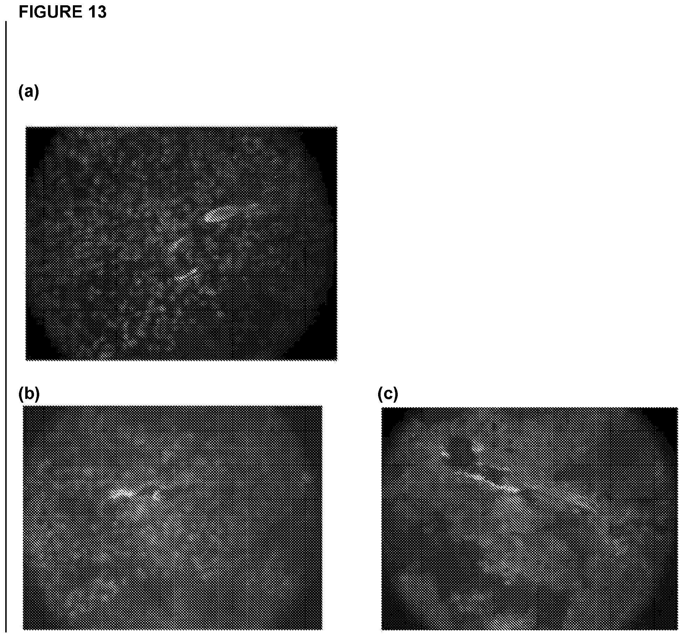

**FIG. 13** shows the effect of ITPP treatment on NK cell invasion of melanoma B16 tumors. B16 tumor cells were labelled with B16F10 DAPI; NK cells were labelled by anti-CD49bFITC1; and vessel endothelial cells were labelled by antiCD31TRITC. (a) untreated B 16 tumor; (b) and (c) B 16 tumor treated with ITPP.

### DETAILED DESCRIPTION

The invention is defined by the appended claims.

### (1) Compositions

Compositions that are useful in accordance with the present invention include acids and salts of inositol trispyrophosphate (ITPP); ITPP is recognized herein as an anion. The term inositol trispyrophosphate, alternatively known as inositol hexaphosphate trispyrophosphate, refers to inositol hexaphosphate with three internal pyrophosphate rings. The counterpart species to ITPP is called a counterion herein, and the combination of ITPP with the counterion is called an acid or salt herein. The invention is not limited to pairings that are purely ionic; indeed, it is well-known in the art that paired ions often evidence some degree of covalent or coordinate bond characteristic between the two components of the pair. The ITPP acids and salts of the invention compositions may comprise a single type of counterion or may contain mixed counterions, and may optionally contain a mixture of anions of which ITPP is one. The compositions may optionally include crown ethers, cryptands, and other species capable of chelating or otherwise complexing the counterions. The compositions may likewise optionally include acidic macrocycles or other species that are capable of complexing the ITPP through hydrogen bonds or other molecular attractions. Methods of making acids and salts of ITPP are described in U.S. Pat. No. 7,084,115 issued to Nicolau et al.

Counterions contemplated for use in the invention include, but are not limited to, the following: cationic hydrogen species including protons and the corresponding ions of deuterium and tritium; monovalent inorganic cations including lithium, sodium, potassium, rubidium, cesium, and copper (I); divalent inorganic cations including beryllium, magnesium, calcium, strontium, barium, manganese (II), zinc (II), copper (II) and iron (II); polyvalent inorganic cations including iron (III); quaternary nitrogen species including ammonium, cycloheptyl ammonium, cyclooctyl ammonium, N₅N- dimethylcyclohexyl ammonium, and other organic ammonium cations; sulfonium species including triethylsulfonium and other organic sulfonium compounds; organic cations including pyridinium, piperidinium, piperazinium, quinuclidinium, pyrrolium, tripiperazinium, and other organic cations; polymeric cations including oligomers, polymers, peptides, proteins, positively charged ionomers, and other macromolecular species that possess sulfonium, quaternary nitrogen and/or charged organometallic species in pendant groups, chain ends, and/or the backbone of the polymer. An exemplary ITPP salt is the monocalcium tetrasodium salt of ITPP or a mixture of sodium ITPP and calcium ITPP that contains 15-25 mol % calcium and 75-85 mol% sodium.

A preferred isomer for the ITPP employed in the present invention is myoinositol, which is cis-1,2,3,5-trans-4,6-cyclohexanehexyl; however, the invention is not so limited. Thus, the invention contemplates the use of any inositol isomer in the ITPP, including the respective tripyrophosphates of the naturally occurring scyllo-, chiro-, muco-, and neo-inositol isomers, as well as those of the allo, epi-, and cis-inositol isomers.

It is contemplated that the ITPP may be formed in vivo from a prodrug, such as by enzymatic cleavage of an ester or by displacement of a leaving group such as a tolylsulfonyl group.

ITPP exhibits anti-angiogenic and anti-tumor properties, and is useful in controlling angiogenesis- or proliferation-related events, conditions or substances. As used herein, the control of an angiogenic- or proliferation-related event, condition, or substance refers to any qualitative or quantitative change in any type of factor, condition, activity, indicator, chemical or combination of chemicals, mRNA, receptor, marker, mediator, protein, transcriptional activity or the like, that may be or is believed to be related to angiogenesis or proliferation, and that results from administering the composition of the present invention.

ITPP also enhances pO₂ in the tumor microenvironment, inhibits metastasis and neoplastic neo-angiogenesis. Hypoxic tumor cells, which are often more invasive and resistant to apoptosis, tend to be resistant to conventional chemotherapy. Thus, in certain embodiments, the efficacy of treatment by chemotherapeutic agent is increased by the combined treatment with ITPP. Further, in some aspects, ITPP treatment induces tumor micro vessel "normalization".

ITPP additionally reduces the number of drug efflux pumps in tumors, and thus, in certain embodiments, treats a cancer resistant to one or more chemotherapeutic agents and/or increases the efficacy of the chemotherapeutic agents against tumor cells.

The present invention provides novel pharmaceutical compositions comprising ITPP and a chemotherapeutic agent. Chemotherapeutic agent suitable for the present invention include: aminoglutethimide, amsacrine, anastrozole, asparaginase, beg, bicalutamide, bleomycin, buserelin, busulfan, camptothecin, capecitabine, carboplatin, carmustine, chlorambucil, cisplatin, cladribine, clodronate, colchicine, cyclophosphamide, cyproterone, cytarabine, dacarbazine, dactinomycin, daunorubicin, dienestrol, diethylstilbestrol, docetaxel, doxorubicin, epirubicin, estradiol, estramustine, etoposide, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, ironotecan, letrozole, leucovorin, leuprolide, levamisole, lomustine, mechlorethamine, medroxyprogesterone, megestrol, melphalan, mercaptopurine, mesna, methotrexate, mitomycin, mitotane, mitoxantrone, nilutamide, nocodazole, octreotide, oxaliplatin, paclitaxel, pamidronate, pentostatin, plicamycin, porfimer, procarbazine, raltitrexed, rituximab, streptozocin, suramin, tamoxifen, temozolomide, teniposide, testosterone, thioguanine, thiotepa, titanocene dichloride, topotecan, trastuzumab, tretinoin, vinblastine, vincristine, vindesine, and vinorelbine.

In one embodiment, the chemotherapeutic agent is a microtubule-targeting agent such as paclitaxel. In another embodiment, the chemotherapeutic agent is a DNA- intercalating agent such as platinum-based agents (e.g., cisplatin) or doxorubicin. In a further embodiment, the chemotherapeutic agent is a nucleoside metabolic inhibitor such as gemcitabine or capecitabine.

In certain embodiments, the chemotherapeutic agent of the composition may be in a subtherapeutic dose or amount. The term "sub-therapeutic dose or amount" means that a dose or amount of a pharmacologically active substance is below the dose or amount of that substance required to be administered, as the sole substance, to achieve a therapeutic effect. The subtherapeutic dose of such a substance will vary depending upon the subject and disease condition being treated, the weight and age of the subject, the severity of the disease condition, the manner of administration and the like, which can readily be determined by one of ordinary skill in the art. In one embodiment, the subtherapeutic dose or amount of the chemotherapeutic agent is less than 90% of the approved full dose of the chemotherapeutic agent, such as that provided in the U.S. Food & Drug Administration-approved label information for the chemotherapeutic agent. In other embodiments, the sub-therapeutic dose or amount of the chemotherapeutic agent is less than 80%, 70%, 60%, 50%, 40%, 30%, 20% or even 10% of the approved full dose, such as from 20% to 90%, 30% to 80%, 40% to 70% or another range within the values provided herein.

The present invention also provides a kit for treating cancer, comprising ITPP and a chemotherapeutic agent. The kit may provide the instructions for using the ITPP and the chemotherapeutic agent in accordance with the treatment regimen or the method of the invention, as discussed below. The chemotherapeutic agent suitable for the kit may include those mentioned above. A sub-therapeutic dose or amount of the chemotherapeutic agent may be used for the kit of the invention.

Also disclosed are implants or other devices comprised of the compounds or drugs of ITPP, or prodrugs thereof, where the drug or prodrug is formulated in a biodegradable or non-biodegradable polymer for sustained release. Non-biodegradable polymers release the drug in a controlled fashion through physical or mechanical processes without the polymer itself being degraded. Biodegradable polymers are designed to gradually be hydrolyzed or solubilized by natural processes in the body, allowing gradual release of the admixed drug or prodrug. The drug or prodrug can be chemically linked to the polymer or can be incorporated into the polymer by admixture. Both biodegradable and non-biodegradable polymers and the process by which drugs are incorporated into the polymers for controlled release are well known to those skilled in the art. Examples of such polymers can be found in many references, such as Brem et al., J. Neurosurg 74: pp. 441-446 (1991). These implants or devices can be implanted in the vicinity where delivery is desired, for example, at the site of a tumor.

Pharmaceutical compositions of this invention may also contain, or be coadministered (simultaneously or sequentially) with, one or more pharmacological agents of value in treating one or more disease conditions referred to hereinabove.

Formulations may generally be prepared and administered according to standard texts, such as Remington's Pharmaceutical Sciences 17^{th}edition. For example, compositions described herein may be formulated in a conventional manner using one or more physiologically or pharmaceutically acceptable carriers or excipients. The compositions of the present invention and their pharmaceutically acceptable salts and solvates may be formulated for administration by, for example, injection (e.g., subcutaneous, intramuscular, intraperitoneal), inhalation or insufflation (either through the mouth or the nose) or oral, buccal, sublingual, transdermal, nasal, parenteral or rectal administration. In one embodiment, a composition may be administered locally, at the site where target cells are present, i.e., in a specific tissue, organ, or fluid (e.g., blood, cerebrospinal fluid, etc.). It should be understood that in addition to the ingredients, particularly those mentioned above, the formulations of the present invention may include other agents conventional in the art having regard to the type of formulation in question, for example, those suitable for oral administration may include flavoring agents or other agents to make the formulation more palatable and more easily swallowed.

Formulations of the present invention suitable for oral administration may be presented as discrete units such as capsules, cachets or tablets, each containing a predetermined amount of the active ingredient; as a powder or granules; as a solution or a suspension in an aqueous liquid or a non-aqueous liquid; or as an oil-in-water liquid emulsion or a water-in-oil emulsion, etc. A tablet may be made by compression or molding, optionally with one or more accessory ingredients. The tablets may optionally be coated or scored and may be formulated so as to provide a slow or controlled release of the active ingredient therein.

Formulations suitable for topical administration in the mouth include lozenges comprising the ingredients in a flavored basis, usually sucrose and acacia or tragacanth; pastilles comprising the active ingredient in an inert basis such as gelatin and glycerin, or sucrose and acacia; and mouthwashes comprising the ingredient to be administered in a suitable liquid carrier.

Formulations suitable for topical administration to the skin may be presented as ointments, creams, gels and pastes comprising the ingredient to be administered in a pharmaceutically acceptable carrier. Another topical delivery system is a transdermal patch containing the ingredient to be administered.

Formulations for rectal administration may be presented as a suppository with a suitable base comprising, for example, cocoa butter and/or a salicylate.

Formulations suitable for nasal administration, wherein the carrier is a solid, include a coarse powder having a particle size, for example, in the range of 20 to 500 microns which is administered in the manner in which snuff is taken; i.e., by rapid inhalation through the nasal passage from a container of the powder held close up to the nose. Suitable formulations, wherein the carrier is a liquid, for administration, as for example, a nasal spray or as nasal drops, include aqueous or oily solutions of the active ingredient.

Formulations suitable for vaginal administration may be presented as pessaries, tampons, creams, gels, pastes, foams or spray formulations containing, in addition to the active ingredient, ingredients such as carriers as are known in the art to be appropriate.

Formulation suitable for inhalation may be presented as mists, dusts, powders or spray formulations containing, in addition to the active ingredient, ingredients such as carriers as are known in the art to be appropriate.

Formulations suitable for parenteral administration include aqueous and nonaqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in freeze-dried (lyophilized) conditions requiring only the addition of a sterile liquid carrier, for example, water for injections, immediately prior to use. Extemporaneous injection solutions and suspensions may be prepared from sterile powders, granules and tablets of the kinds previously described.

Formulations contemplated as part of the present invention include nanoparticles formulations made by methods disclosed in U.S. Publication No. 2004/0033267. The particles of the compounds of the present invention have an effective average particle size of less than about 2 microns, less than about 1500 nm, less than about 1000 nm, less than about 500 nm, less than about 250 nm, less than about 100 nm, or less than about 50 nm, as measured by light-scattering methods, microscopy, or other appropriate methods well known to those of ordinary skill in the art.

### (2) Treatment Regimen and Method

ITPP induces intratumor vascular normalization. ITPP-induced vascular normalization counteracts tumor hypoxia, a key reason for tumor cells' resistance to both radiation and cytotoxic drugs and for tumor metastasis.

In one aspect, disclosed is a treatment regimen or a method for treating cancer or tumors in a subject that includes administering simultaneously or sequentially a therapeutically effective amount of ITPP and a chemotherapeutic agent. The phrase "therapeutically effective amount" means that amount of such a substance, composition, kit or treatment regimen as a whole that produces some desired local or systemic effect, typically at a reasonable benefit/risk ratio in the context of a treatment regimen or method. The therapeutically effective amount of such substance will vary depending upon the subject and disease condition being treated, the weight and age of the subject, the severity of the disease condition, the manner of administration and the like, which can readily be determined by one of ordinary skill in the art. For example, certain compositions described herein may be administered in a sufficient amount to produce a desired effect at a reasonable benefit/risk ratio applicable to such treatment.

Suitable chemotherapeutic agents suitable to be used in the methods may include those mentioned above. The chemotherapeutic agent is paclitaxel, cisplatin or gemcitabine.

Exemplary cancers include, but are not limited to, haematologic neoplasms, including leukaemias, myelomas and lymphomas; carcinomas, including adenocarcinomas and squamous cell carcinomas; melanomas and sarcomas. Carcinomas and sarcomas are also frequently referred to as "solid tumors." Types of tumors that may be treated by the methods of the present invention are preferably solid tumors including, but not limited to: sarcomas, carcinomas and other solid tumor cancers, including, but not limited to germ line tumors, tumors of the central nervous system, breast cancer, prostate cancer, cervical cancer, uterine cancer, lung cancer, ovarian cancer, testicular cancer, thyroid cancer, astrocytoma, glioma, pancreatic cancer, stomach cancer, liver cancer, colon cancer, melanoma, renal cancer, bladder cancer, esophageal cancer, cancer of the larynx, cancer of the parotid, cancer of the biliary tract, rectal cancer, endometrial cancer, squamous cell carcinomas, adenocarcinomas, small cell carcinomas, neuroblastomas, mesotheliomas, adrenocortical carcinomas, epithelial carcinomas, desmoid tumors, desmoplastic small round cell tumors, endocrine tumors, Ewing sarcoma family tumors, germ cell tumors, hepatoblastomas, hepatocellular carcinomas, lymphomas, melanomas, nonrhabdomyosarcome soft tissue sarcomas, osteosarcomas, peripheral primative neuroectodermal tumors, retinoblastomas, rhabdomyosarcomas, and Wilms tumors.

In one embodiment, ITPP and the chemotherapeutic agent are administered simultaneously. In a specific embodiment, ITPP and a nucleoside metabolic inhibitor such as gemcitabine, the chemotherapeutic agent, are administered simultaneously. In another specific embodiment, the cancer is pancreatic cancer. In certain embodiments, the cancer is melanoma.

In another embodiment, ITPP and the chemotherapeutic agent are administered sequentially. For example, ITPP is administered prior to the administration of the chemotherapeutic agent. In a preferred embodiment, the chemotherapeutic agent is administered after the occurrence of partial vascular normalization in the tumor. As used herein "partial vascular normalization" refers to a physiological state during which existing tumor vasculature exhibits improved structure in the vascular endothelium and basement membrane and therefore have reduced leakiness, dilation and/or hypoxia. Such partial vascular normalization may be determined by detecting and/or monitoring the change in the level of one or more of pO₂, hypoxia-inducible factor 1 alpha (HIF-1α), VEGF, tyrosine kinase Tie-2, and hemo-oxygenase 1 (HO-1), or by monitoring the physiological state of the tumor vessels using technologies including Magnetic Resonance Imaging (MRI) and Magnetic Resonace Angiography (MRA).

In a preferred embodiment, ITPP is administered about 2 hours to 5 days prior to the administration of the chemotherapeutic agent. In another preferred embodiment, ITPP is administered about 1 to 4 days prior to the administration of the chemotherapeutic agent, such as 2 to 3 days prior to the administration of the chemotherapeutic agent (e.g., a microtubule-targeting agent such as paclitaxel or a DNA intercalator such as cisplatin).

Multiple rounds of ITPP and a chemotherapeutic agent can be administered. In certain embodiments, only one round is administered. In other embodiments, two or more rounds (e.g., two, three, four, or more rounds) of ITPP and the chemotherapeutic agent are administered. The rounds may be separated by 1 day to 6 months, such as from 1 day to 3 months, 1 week to 2 weeks, 2 weeks to 3 weeks, 3 weeks to 1 month, 1 month to 2 months, or 2 months to 3 months.

The chemotherapeutic agent may be administered in a sub-therapeutic dose or amount, based upon the dose for that agent as a sole active agent. In one embodiment, the sub-therapeutic dose or amount of the chemotherapeutic agent administered is less than 90% of the approved full dose of the chemotherapeutic agent or another dose as described above.

Disclosed is a method of treating a drug resistant cancer. In certain embodiments, a drug resistant cancer is a cancer that is not treatable with one or more chemotherapeutic agents. For example, a drug resistant cancer may have no appreciable reduction in tumor size upon treatment with an agent and/or does not significantly inhibit progression of a tumor (e.g., from Stage II to Stage III or from Stage III to Stage IV). Examples of chemotherapeutic agents that certain cancers, particularly melanoma, are resistant to include microtubule-targeting agents (e.g., paclitaxel) and DNA intercalators (e.g., platinum-based ones such as cisplatin). Drug resistance assays are described in, for example, Lowe et al. (1993) Cell 74:95 7-697. A drug resistant cancer is a cancer having significantly increased levels of canalicular multispecific organic anion transporter 1 and/or the P-glycoprotein drug efflux pump as compared to a non-resistant cancer cell.

Methods of treatment a drug resistant cancer can involve either administration of ITPP alone or of ITPP in combination with another chemotherapeutic agent, such as those described herein.

Disclosed are also methods for treating a hyper-proliferative condition comprising administering to a subject in need thereof a therapeutically effective amount of ITPP, wherein the hyper-proliferative condition is not cancer or characterized by undesired angiogenesis. Hyper-proliferative conditions that may be treated by the disclosed methods include, but not limited to: diabetic nephropathy, glomerulosclerosis, IgA nephropathy, cirrhosis, biliary atresia, congestive heart failure, scleroderma, radiation-induced fibrosis, lung fibrosis (idiopathic pulmonary fibrosis, collagen vascular disease, sarcoidosis, interstitial lung diseases and extrinsic lung disorders), psoriasis, genital warts and hyperproliferative cell growth diseases, including hyperproliferative keratinocyte diseases such as hyperkeratosis, ichthyosis, keratoderma or lichen planus. The tissue or organ displaying the hyperproliferative condition is hypoxic. The method for treating a hyper-proliferative condition further comprises administering an additional antihyperproliferative agent. Antihyperproliferative agents include doxorubicin, daunorubicin, mitomycin, actinomycin D, bleomycin, cisplatin, VP16, an enedyine, taxol, vincristine, vinblastine, carmustine, mellphalan, cyclophsophamide, chlorambucil, busulfan, lomustine, 5-fluorouracil, gemcitabin, BCNU, or camptothecin.

Disclosed is further a method for enhancing immune response in a subject, comprising administering to a subject in need thereof a therapeutically effective amount of ITPP, wherein the subject does not suffer from cancer or another tumor. In one embodiment, the subject does not suffer from undesired angiogenesis.

### (3) Definitions

As used herein, the following terms and phrases shall have the meanings set forth below. Unless defined otherwise, all technical and scientific terms used herein have the same meaning as commonly understood to one of ordinary skill in the art.

The term "agent" is used herein to denote a chemical compound, a mixture of chemical compounds, a biological macromolecule (such as a nucleic acid, an antibody, a protein or portion thereof, e.g., a peptide), or an extract made from biological materials such as bacteria, plants, fungi, or animal (particularly mammalian) cells or tissues. The activity of such agents may render it suitable as a "therapeutic agent" which is a biologically, physiologically, or pharmacologically active substance (or substances) that acts locally or systemically in a subject.

The terms "parenteral administration" and "administered parenterally" are art- recognized and refer to modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intra-articular, subcapsular, subarachnoid, intraspinal, and intrasternal injection and infusion.

A "patient", "subject", "individual" or "host" refers to either a human or a non-human animal.

A "cytotoxic drug or agent" is any agent capable of destroying cells, preferably cancer cells.

The term "pharmaceutically acceptable carrier" is art-recognized and refers to a pharmaceutically-acceptable material, composition or vehicle, such as a liquid or solid filler, diluent, excipient, solvent or encapsulating material, involved in carrying or transporting any subject composition or component thereof. Each carrier must be "acceptable" in the sense of being compatible with the subject composition and its components and not injurious to the patient. Some examples of materials which may serve as pharmaceutically acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) phosphate buffer solutions; and (21) other non-toxic compatible substances employed in pharmaceutical formulations.

The term "therapeutic agent" is art-recognized and refers to any chemical moiety that is a biologically, physiologically, or pharmacologically active substance that acts locally or systemically in a subject. The term also means any substance intended for use in the diagnosis, cure, mitigation, treatment or prevention of disease or in the enhancement of desirable physical or mental development and/or conditions in an animal or human.

"Treating" a condition or disease refers to curing as well as ameliorating at least one symptom of the condition or disease. Treating includes administration of a composition which reduces the frequency of, or delays the onset of, symptoms of a medical condition in a subject relative to a subject which does not receive the composition. Thus, treatment of cancer includes, for example, reducing the number and/or size of detectable cancerous growths in a population of patients receiving a treatment relative to an untreated control population, and/or delaying the appearance of detectable cancerous growths in a treated population versus an untreated control population, e.g., by a statistically and/or clinically significant amount.

### EXEMPLIFICATION

The invention now being generally described, it will be more readily understood by reference to the following examples which are included merely for purposes of illustration of certain aspects and embodiments of the present invention.

Examples not pertaining to the invention are for illustrative purposes only.

### Example 1 Production of B16F10LucGFP cell line

B16F10 murine melanoma transduction was done with retroviral vectors: firefly luciferase cDNA driven by 5'LTR promoter then IRES sequence and EGFP cDNA. The vectors were produced using a pBMN-Luc-I-GFP plasmid (kindly gifted by Dr. Magnus Essand, Uppsala, Sweden) and a PT67 packaging cell line (Clontech) stably expressing the *gag*, *pol,* and *env* genes. Additionally, the pM13 plasmid providing *gag* and *pol* gene (kindly gifted by Dr. Christine Brostjan, Vienna, Austria) was used to increase the production efficiency. The packaging cells were cultured in DMEM HG medium (PAA Laboratories) supplemented with 10% FCS, penicillin (100 U/mL) and streptomycin (100 µg/mL), and co-transfected with pBMN-Luc-I-EGFP and pM13 plasmids using SuperFect reagent (Qiagen) according to manufacturer instruction. After transfection the cells were cultured at 32 C for 48 h. Then media containing the retroviral vectors were collected, mixed with complete RPMI medium in the v/v ratio of 1:1 and used for transduction of B16F10 cells. Three days later the transduction efficiency was estimated using fluorescent microscope, according to the presence of EGFP (about 5%). After several passages of EGFP positive colonies and three sets of sorting using the MoFlo Flow Cytometer (Dako Cytomation) the B16F10LucGFP cell line of more than 99% purity was obtained.

### Example 2 Cell Susceptibility to Chemotherapy According to O₂ Pressure

Dose response curves to cisplatin (*cis*-dichlorodiamine platinum) (Sigma- Aldrich) or paclitaxel (Calbiochem) were established under various pO2 % for 48h. Cell viability was evaluated by Alamar blue test (Biosource) as described by the manufacturer.

### Example 3 Experimental Metastasis Assay

After intravenous injection of B16F10LucGFP murine melanoma cells (10⁵ cells in 0.1 ml saline) in the tail vein, mice (eight- week-old female C57BL/6 from Janvier) were treated by 1,5 g/kg ITPP IP injected every 5 days (10 mice per treatment group). Treatment was initiated at day 5 after tumor cells inoculation. 19 or 27 days later, mice were euthanized and lungs collected separately. Macroscopic lung foci were counted and luciferase was determined by chemoluminescence assay (Promega) in order to quantify the amount of melanoma cells in tissues. All animal study procedures and the use of animals were approved by the Comite d'Ethique pour l'expérimentation animale, Campus CNRS d'Orléans, France.

### Example 4 Subcutaneous Melanoma Model

B16F10LucGFP cells were grown as subcutaneous tumors, after injecting 100 µl of a plug constituted by 10⁵ cells in 25% MatrigelTM (50% in OptiMEM). Matrigel was from BD Biosciences and OptiMEM from Invitrogen. Mice were euthanized and tumors and lungs excised 25 days after inoculation. Various protocols of treatments were applied according to the time and dose of ITPP (100 µg/kg to 2.0 g/kg in saline, intraperitoneally) in combination with cisplatin (10 mg/kg in saline, intraperitoneally) and paclitaxel (2 mg/kg, in 50% ethanol 50% Cremophor EL; Sigma-Aldrich, per os). Treatment was initiated 7, 9 or 11 days post melanoma cell inoculation.

### Example 5 Biochemical Quantification of the Hypoxic-, Angiogenesis- and

### Melanoma-related markers

Lungs were homogenized in lysis buffer (Active motif). After centrifugations, clear supernatant was collected. Total protein amount was determined by BCA protein assay kit (Thermo Scientific). HIF-1α, VEGF, Tie-2 and HO-1 in lung lysates were quantified using colorimetric sandwich ELISA according to the manufacturer's instructions. The HIF- 1α, VEGF and Tie-2 ELISA kits were from R&D. The HO- 1 ELISA kit was from Takara.

### Example 6 Semi Quantitative Reverse Transcriptase Polymerase Chain Reaction Analysis

The Taqman polymerase chain reaction primer sequences for LOX were 5'-ATCGCCACAGCCTCCGCAGCTCA-3' (SEQ ID NO: 1) and 5'-AGTAACCGGTGCCGTATCCAGGTCG-3' (SEQ ID NO: 2). For β-actin (internal control), the primer sequences were 5'- CCAGAGCAAGAGAGGC ATCC-3' (SEQ ID NO: 3) and 5'-CTGTGGTGGTGAAGCTGAAG-3' (SEQ ID NO: 4). The amplified cDNAs bands were quantified with ImageQuant software (Becton and Dickinson). LOX mRNA levels were normalized relatively to β-actin mRNA.

### Example 7 Immunohistological Staining

Tumor tissues were embedded in Tissue-Tek (Sakura), tissue freezing medium and snap frozen in liquid nitrogen. Cryosections were fixed and stained using a rat monoclonal IgG2a antibodies against mouse CD31 (PECAM-I, platelet/endothelial cell adhesion molecule) from eBiosciences, rabbit IgG anti-SMA (smooth muscle actin) antibody (AbCAM), or mouse IgG2a anti-P-Glycoprotein (C219) (Calbiochem), diluted 1 :200 in FCS 5% in PBS. Goat IgG-FITC-labelled anti-rat immunoglobulin, goat IgG- FITC-labelled anti-rabbit immunoglobulin or goat IgG-FITC-labelled anti-mouse immunoglobulin (diluted 1 :200 in PBS) was used as secondary antibodies respectively. To detect cell nuclei, sections were incubated with bis-Benzimide H 33258 (Sigma- Aldrich) 1:1000 in PBS. Specimens were mounted in Vectashield (Vector) and fluorescent microscopy detection was performed on a Zeiss 200M inverted fluorescent microscope. Tumor necrosis was analysed after Hematoxylin eosine staining of tumor sections.

### Example 8 Magnetic Resonance Imaging (MRI)

MRI assays were performed with a 9.4 T horizontal magnet for small animals (94/21 USR Bruker Biospec), equipped with a 950mT/m gradient set. Mice were placed in a linear homogeneous coil (inner diameter: 35 mm). Animals were maintained under gaseous (50% N₂O: 0.7 1/min - 50% O₂: 0.71/min - Isoflurane 1.5%) anesthesia, temperature kept constant at 36°C and breathing rate was monitored during the acquisitions using air balloon placed on the mouse chest to adjust the anesthetic output. Measurement of tumor vascularization was performed by MR angiography using the Fast Low Angle Shot (FLASH) sequence both in the axial and coronal planes. The FLASH pulse sequence was adapted to the study of the evolution of the angiogenesis of the tumor. This technique allows the 3D structure of the vascular tree of the tumor on the same animal to be followed overtime.

### Example 9 Oxylite pO₂ Measurement

The oxylite 2000E P_{O2} system (Oxford Optronics) measures pO₂ by determining the O₂-dependant fluorescence lifetime of ruthenium chloride which is immobilized at the tip of 230-µm diameter fiber-optic probe. The lifetime of the fluorescent pulse is inversely proportional to the oxygen tension in the tip. The mouse was anesthetized with intraperitoneal injection of xylazine/ketamin before the oxylite probe tip was installed inside the tumor and oxygen pressure recorded as described.

### Example 10 ITPP-RBCs Selectively Counteract Hypoxia in the Tumor Microenvironment

To confirm that ITPP-RBCs counteract hypoxia *in vivo,* a comparison of the values of the oxygen tension inside the melanoma tumors implanted subcutaneously above the left leg, in ITPP- treated and untreated mice was performed. Oxygen pressure (p02) was computed by determining the O₂-dependent fluorescence lifetime of ruthenium chloride on the tip of a fibre - optic probe. The lifetime of the fluorescent pulse is inversely proportional to the oxygen tension in the tip of the probe. While the tumors in non-treated animals, were strongly hypoxic with an oxygen pressure value below 2 mmHg (Fig. 1), in tumors of ITPP-treated mice pO2 reached the range of 40 mmHg (Fig. 1A). This pO₂increase occurred as soon as 30 minutes after intraperitoneal injection of ITPP (Fig. 1B, C) and was maintained at a high level, up to 40 mmHg, as shown 24 hours after injection (Fig. 1A) for at least 48 hours. Moreover, ITPP-RBCs targeted specifically the hypoxic tumors since, as shown in the muscle, of the corresponding non tumoral (right) leg of the same animal, no change or any effect on pO₂ was detected in parallel and concomitant measurements (Fig. 1C) while in the tumor the pO2 level was increased 30 min after ITPP injection.

### Example 11 ITPP-RBCs Prevent Lung Metastases Formation by B16 Melanoma Cells

To validate ITPP as an anti-metastatic agent, the "artificial" model of lung metastasis was used by injecting intravenously melanoma cells in mice. The B16F10LucGFP cell line was used, which is the melanoma B16F10 line transduced by GFP and luciferase reporter genes allowing to track and quantify the melanoma cells by analysis of luciferase activity in tissues. Experiments comparing the biological behaviour of the B16F10 melanoma cell line to the B16F10LucGFP cells, in terms of proliferation angiogenesis promotion and metastatic development, showed no significant differences and, no sensitivity of the luciferase to hypoxia thus validating their use.

In vivo experiments were pursued until 27 days after inoculation of melanoma cells. Metastatic nodules were very significantly reduced when ITPP treatment started from day 5 after B16 cells injection. This effect could be quantified by measuring the luciferase activity in the lungs (Fig.2A). It allowed biochemical quantification of micrometastases, undetectable by visual examination. To investigate whether the ITPP effect was associated with changes in oxygen partial pressure in the nodules, expression of the HIF- 1α isoform of the hypoxia-inducible factor-α subunit was analyzed, which is crucial for the response of mammalian cells to oxygen levels and is considered to be the cellular O₂ sensor. Once bound to the Hypoxia Response Element (HRE), it turns on the hypoxia-related gene cascade. Fig.2B shows that HIF- 1α levels, which were clearly upregulated in lungs of untreated melanoma bearing mice, decreased dramatically in lungs of ITPP-treated mice.

The vascular endothelial growth factor (VEGF) level is dependent upon HIF- 1α and the main target for anti-angiogenic treatments, decreased to control levels (Fig. 2C), as assayed by ELISA, under the influence of ITPP-RBCs. These results were confirmed by studies on Tie-2 expression. Tie-2 is a specific endothelial tyrosine kinase receptor, essential for the maturation of normal blood vessels which declines in hypoxia. This marker, which is significantly reduced in hypoxic lungs, was re -induced by ITPP treatment (Fig. 2D), indicating that - the vessels in metastatic nodules were submitted to disorganized angiogenesis and, when angiogenesis was regulated by ITPP-RBCs, the more mature vessels re-expressed the Tie-2 marker. After ITPP treatment, heme oxygenase- 1 (HO-1), a cytoprotective enzyme induced by HIF- 1α, was also significantly reduced compared to non-treated mice (Fig. 2E). The over-expression of HO-1 increases viability, proliferation of cells and angiogenic potential of melanoma cells, augments metastasis, and decreases survival of control, tumor-bearing mice. Additional studies on the semiquantitative PCR mRNA analysis of lysyl-oxidase (Fig. 2F), an enzyme involved in the invasive process of cancer cells and which is hypoxically regulated, also demonstrated the beneficial effect of ITPP treatment, resulting in "low O₂ -affinity RBCs".

### Example 12 ITPP-RBCs Eradicate Orthotopic Melanoma Lung Metastases

The effects of ITPP-RBCs on metastasis were assessed following subcutaneous primary tumor implantation. In short-term treatment (3 ITPP injections, 5 days intervals) started on day 7 post tumor inoculation, ITPP reduced significantly the lung metastases (Fig.3A). Initiation of ITPP treatment at day 7 was optimal both in short-term and chronic administration protocols (days 7, 12, 16, 18, 19). Initiation on day 9 or 11 was less effective, chronic administrations even resulted in enhancement of pulmonary metastases (Fig. 3A). Although the reason for this observation is not clear, chronic administration leading to a complete inhibition of angiogenesis could additionally alter the phenotype of tumors, increasing invasiveness and metastasis.

### Example 13 ITPP-RBCs Induce Intratumor Vessel Normalization

Structural changes of microcirculation in tumors were assessed by MRA (Magnetic Resonance Angiography) adaptation of Magnetic Resonance Imaging (MRI) to follow the 3D structure of the vascular tree of the tumor.

21 days after melanoma development, the tumors displayed a typical chaotic vessel architecture (Fig. 3Ba). In mice treated with ITPP on day 9 and 14, the vasculature became less dense and remarkably normalized after additional repeated treatments on days 18 and 19. Intra-tumoral examination revealed numerous vessels at the periphery; normalization was shown by recruitment of pericytes surrounding the vessels and labelled by anti smooth muscle antigen antibodies (Fig. 3Bb) while no such ordering appeared in the non treated tumor (Fig.3Ba,b). This tendency toward "normalization" was accompanied by a remarkable reduction of the tumor size (Fig. 3Ba).

In the same implanted primary tumors, the effect of ITPP treatment on the multidrug efflux pumps responsible for drug resistance were examined. ITPP down- regulates the P-glycoprotein drug efflux pump. This effect may counteract the chemo resistance and the failure of drug-target interactions, due to a reduction of the effective intracellular concentration of the drug. Thus, the ITPP-induced normalization of vessels correlates with the reduction of drug efflux pumps in tumors, and thus may increase the efficacy of the drugs against tumor cells.

### Example 14 ITPP-RBCs Eradicate Orthotopic Melanoma Lung Metastases and Metronomically Synergizes Chemotherapy

Because of the ability of ITPP to improve oxygen delivery to hypoxic tissues by RBCs, its effect on the efficacy of melanoma treatment by drugs such as paclitaxel and cisplatin was studied. The effects of the drugs on B16 melanoma cells in normoxia, hypoxia and after re-oxygenation were first tested in vitro. Fig. 4 shows that drug cytotoxicity towards B16 cells decreased as oxygen tension decreased (1% or 11%). However, upon re-oxygenation (from 1% to 11% and 20%) of the cells, the cytotoxicity of the drugs was re-established to an extent dependent on the pO2 level (Fig.4A, B). These data compared to the in vivo modulation of the p-glycoprotein in the tumor cells suggest that the sensitivity to drugs which is controlled by the hypoxia-induced enhancement of the MDR could be reversed by the ITPP induced reoxygenation of the tumor.

ITPP treatment was combined with paclitaxel and cisplatin in vivo. The lung metastases dramatically increased (Fig. 5A) after simultaneous treatment with ITPP, paclitaxel and cisplatin with a profile similar to that observed with chronic treatment with ITPP alone (see above). Paclitaxel and cisplatin by themselves inhibited the growth of the endothelial cells (Fig. 4C), supporting an anti-angiogenic effect of these compounds *in vivo.* Such anti-angiogenic treatments that destroy tumor vascularisation have been found to enhance metastatic invasion by selection of hypoxia resistant tumor cells thus corroborating the data shown on Fig. 3 and indicating that vessel normalization, rather than disruption or elimination of tumor neo-angiogenesis, is believed to be a more relevant and potentially beneficial approach to cancer therapy.

The effect of ITPP- and drug-injection schedule on the treatment of both developed solid tumors (Fig 5B) and lung metastases (Fig.5A) was tested. Mice, treated with ITPP alone until day 14, were exposed again to ITPP alone on days 18 and 19, in the attempt to normalize the vessels, followed by cisplatin plus paclitaxel treatments on days 20 and 21 before analysis on day 25. The results were spectacular: the lung metastases were eradicated, in direct contrast with simultaneous treatment (Fig. 5A), but confirming the importance of the metronomic parameter in the protocol setting for certain cancer therapies. Indeed, analysis of tumor microvessels by CD31 (PECAM-1) staining, which is a specific marker of endothelium, displayed, after treatment with the chemotherapeutic drugs, a reduced density of intratumor microvessels in ITPP-treated animals compared to the large numbers of poorly structured microvessels with irregular shape and prominent CD31 staining of the endothelial cells in controls (Fig. 5Ba). Moreover, it is shown in Fig. 5B that, by prolonging regular treatment by ITPP at days 18 and 19, thus aiming to normalize the vessels and the oxygen tension prior to the drug treatment on days 20 and 21, the cytoxicity was strongly enhanced, as indicated by the necrosis on day 25 showing the necrotic areas that correspond to diffuse CD31 positivity (Fig. 5Ba3) and that are delineated by H&E staining (Fig. 5Ba3 and Fig.5Bb3) and confirmed by the tumor size reduction and necrosis induction. This points to the strong effect of the ITPP treatment combined with chemotherapy.

### Example 15 ITPP In Combination with Gemcitabine Treatment Shows Strong Additive Effects in Animal Models

In both rat pancreatic tumor model and Human Panc-1 pancreatic tumor xenograft mice model, ITPP in combination with gemcitabine treatment showed a strong additive effect. The effect of ITPP treatment alone was first examined on both models, as compared to the effect of gemcitabine and placebo (Figures 6 and 8). The effect of ITPP in combination with gemcitabine treatment on both models was then investigated, as compared to the effect of gemcitabine treatment alone and placebo.

In the rat pancreatic tumor model, rats in the combination treatment group received ITPP (1.5 mg/Kg) in combination with gemcitabine (25 mg/Kg or 50 mg/Kg) weekly during the period of day 14 to day 49. Rats in the gemcitabine treatment group received administration of gemcitabine (100 mg/Kg) alone on days 16, 18 and 20. Rats in the control group were not treated. The survival rate of the tested animals were significantly enhanced in the combination treatment group. The animal survival profile also demonstrated a dose dependency on gemcitabine (Figure 7).

In the xenograft tumor model, mice in the combination treatment group received administration of ITPP (2 mg/Kg) in combination with gemcitabine (25 mg/Kg or 50 mg/Kg) weekly during the period of day 14 to day 49. Mice in the gemcitabine treatment group received administration of gemcitabine (100 mg/Kg) on days 16, 18 and 20. Mice in the control group were not treated. It was shown that the combination treatment enhanced the animal survival index, as compared to gemcitabine treatment alone, albeit without any dose dependency on gemcitabine (Figure 9).

Median survival time of animals with ductal pancreatic adenocarcinoma treated with OXY111A and/or gemcitabine was followed and summarized in Table 1 below:

| Model | OXY111A alone | Gemcitabine alone | OXY111A + Gemcitabine | Untreated control |
|---|---|---|---|---|
| Syngeneic rat tumor in rat | 102 d (1.5 mg/kg) | 58 d (100 mg/kg) | >300 d | 45 d |
| Rat tumor in Nude Mouse | 107 d (2 mg/kg) | 89 d (100 mg/kg) | 76 d (50 mg/kg) | 69 d |
| | | | 102 d (25 mg/kg) | |
| Human Panc-I in Nude Mouse | 155 d (2 mg/kg) | 141 d (100 mg/kg) | 150 d (50 mg/kg) | 127 d |
| | | | 154 d (25 mg/kg) | |
| Human MiaPaca in Nude Mouse | 155 d (2 mg/kg) | 140 d (100 mg/kg) | n/a | 75 d |

In the rat pancreatic tumor model, the expression of HIF-1α, VEGF, caspase-3 and β-actin were further examined following the treatment of ITPP, gemcitabine or placebo (Figure 10).

### Example 16 ITPP Treatment Enhances Immune Cell Infiltration into and Invasion of Tumors

In a B16 tumor model, it was demonstrated that OXY111A treatment significantly enhanced the infiltration of CD68 (M2 type) macrophages into a B16 tumor after intraperitoneal injections of OXY111A on days 7,8, 14, 15, 21,22, 29 and 30 (Figure 11).

In the same model, ITPP treatment also significantly enhanced the infiltration of the CD49b NK cells and the presence of CD31 EC cells in a B16 tumor, and the invasion of the NK cells in melanoma B16 tumors (Figures 12 and 13).

## Claims

1. A therapeutically effective amount of inositol trispyrophosphate (ITPP) for use in partial vascular normalization of a tumor in a cancer patient prior to the administration of an effective amount of a chemotherapeutic agent, wherein the chemotherapeutic agent is:
a microtubule-targeting agent selected from colchicine, docetaxel, paclitaxel, vinblastine, vincristine, vindesine, and vinorelbine;
a DNA-intercalating agent selected from cisplatin, daunorubicin, doxorubicin, or epirubicin; or
a nucleoside metabolic inhibitor selected from cytarabine, fludarabine, fluorouracil, gemcitabine, mercaptopurine, or pentostatin.

2. A therapeutically effective amount of ITPP for use according to claim 1, further comprising detecting the occurrence of partial vascular normalization in the tumor.

3. A therapeutically effective amount of ITPP for use according to claim 2, wherein the occurrence of partial vascular normalization is detected by measuring partial oxygen pressure (pO₂) level of the tumor.

4. A therapeutically effective amount of ITPP for use according to claim 1, wherein the chemotherapeutic agent is administered in a sub-therapeutic dose.

5. A therapeutically effective amount of ITPP for use according to claim 1, wherein the cancer is resistant to one or more chemotherapeutic agents and efficacy of the chemotherapeutic agents against tumor cells is increased.

6. A therapeutically effective amount of ITPP for use according to claim 1, wherein the chemotherapeutic agent is a microtubule-targeting agent that is selected from docetaxel and paclitaxel.

7. A therapeutically effective amount of ITPP for use according to claim 1, wherein the chemotherapeutic agent is DNA-intercalating agent that is selected from platinum-based agents and doxorubicin.

8. A therapeutically effective amount of ITPP for use according to claim 7, wherein the platinum-based agent is cisplatin.

9. A therapeutically effective amount of ITPP for use according to claim 5, wherein cisplatin is combined with paclitaxel in the treatment of melanoma and lung metastasis.

10. A therapeutically effective amount of ITPP for use according to claim 1, wherein the chemotherapeutic agent is a nucleoside metabolic inhibitor that is selected from gemcitabine and capecitabine.

11. A therapeutically effective amount of ITPP for use according to claim 1, wherein ITPP is administered about 2 hours to 5 days prior to the administration of the chemotherapeutic agent.

12. A therapeutically effective amount of ITPP according for use to claim 1, wherein the cancer is selected from solid tumors including sarcomas, carcinomas, germ line tumors, tumors of the central nervous system, breast cancer, prostate cancer, cervical cancer, uterine cancer, lung cancer, ovarian cancer, testicular cancer, thyroid cancer, astrocytoma, glioma, pancreatic cancer, stomach cancer, liver cancer, colon cancer, melanoma. renal cancer, bladder cancer, esophageal cancer, cancer of the larynx, cancer of the parotid, cancer of the biliary tract, rectal cancer, endometrial cancer, squamous cell carcinomas, adenocarcinomas, small cell carcinomas, neuroblastomas, mesotheliomas, adrenocortical carcinomas, epithelial carcinomas, desmoid tumors, desmoplastic small round cell tumors, endocrine tumors, Ewing sarcoma family tumors, germ cell tumors, hepatoblastomas, hepatocellular carcinomas, lymphomas, melanomas, nonrhabdomyosarcome soft tissue sarcomas, osteosarcomas, peripheral primative neuroectodermal tumors, retinoblastomas, rhabdomyosarcomas, and Wilms tumors.

13. A therapeutically effective amount of ITPP for use according to claim 12, wherein the cancer is melanoma.

14. A therapeutically effective amount of ITPP for use according to claim 12, wherein the cancer is pancreatic cancer.

15. A kit for treating solid tumors and reducing metastasis comprising inositol trispyrophosphate (ITPP) and a chemotherapeutic agent, wherein the chemotherapeutic agent is a microtubule targeting agent, a DNA-intercalating agent, or a nucleoside metabolic inhibitor.

16. A kit according to claim 15, wherein the chemotherapeutic agent is a microtubule-targeting agent that is selected from docetaxel and paclitaxel.

17. A kit according to claim 15, wherein the chemotherapeutic agent is the DNA-intercalating agent that is selected from platinum-based agents and doxorubicin.

18. A kit according to claim 17, wherein the platinum-based agent is cisplatin.

19. A kit according to claim 18, wherein the cisplatin is combined with paclitaxel.

20. A kit according to claim 15, wherein the chemotherapeutic agent is nucleoside metabolic inhibitor that is selected from gemcitabine and capecitabine.

21. A kit according to claim 15, wherein the kit further provide the instructions for administering a therapeutically effective amount of inositol trispyrophosphate (ITPP) in a cancer patient prior to the administration of an effective amount of said chemotherapeutic agent.

22. A therapeutically effective amount of ITPP for use according to claim 1, wherein ITPP inhibits metastasis.

## Patentansprüche

1. Eine therapeutisch wirksame Menge an Inositoltrispyrophosphat (ITPP) zur Verwendung bei der partiellen vaskulären Normalisierung eines Tumors bei einem Krebspatienten vor der Verabreichung einer wirksamen Menge eines Chemotherapeutikums, wobei das Chemotherapeutikum:
ein Mikrotubuli-Targeting-Agens, ausgewählt aus Colchicin, Docetaxel, Paclitaxel, Vinblastin, Vincristin, Vindesin und Vinorelbin;
ein DNA-Interkalations-Agens, ausgewählt aus Cisplatin, Daunorubicin, Doxorubicin oder Epirubicin; oder
ein Inhibitor des Nukleosidmetabolismus, ausgewählt aus Cytarabin, Fludarabin, Fluoruracil, Gemcitabin, Mercaptopurin oder Pentostatin ist.

2. Eine therapeutisch wirksame Menge an ITPP zur Verwendung nach Anspruch 1, ferner umfassend das Erfassen des Auftretens einer partiellen vaskulären Normalisierung in dem Tumor.

3. Eine therapeutisch wirksame Menge an ITPP zur Verwendung nach Anspruch 2, wobei das Auftreten einer partiellen vaskulären Normalisierung durch Messen des partiellen Sauerstoffdrucks (pO₂) des Tumors nachgewiesen wird.

4. Eine therapeutisch wirksame Menge an ITPP zur Verwendung nach Anspruch 1, wobei das Chemotherapeutikum in einer subtherapeutischen Dosis verabreicht wird.

5. Eine therapeutisch wirksame Menge an ITPP zur Verwendung nach Anspruch 1, wobei der Krebs gegen ein oder mehrere Chemotherapeutika resistent ist und die Wirksamkeit der Chemotherapeutika gegen Tumorzellen erhöht ist.

6. Eine therapeutisch wirksame Menge an ITPP zur Verwendung nach Anspruch 1, wobei das Chemotherapeutikum ein Mikrotubuli-Targeting-Agens ist, das aus Docetaxel und Paclitaxel ausgewählt ist.

7. Eine therapeutisch wirksame Menge an ITPP zur Verwendung nach Anspruch 1, wobei das Chemotherapeutikum ein DNA-Interkalations-Agens ist, das aus Platin-basierten Agenzien und Doxorubicin ausgewählt ist.

8. Eine therapeutisch wirksame Menge an ITPP zur Verwendung nach Anspruch 7, wobei das Platin-basierte Agens Cisplatin ist.

9. Eine therapeutisch wirksame Menge an ITPP zur Verwendung nach Anspruch 5, wobei Cisplatin mit Paclitaxel bei der Behandlung von Melanom und Lungenmetastasen kombiniert wird.

10. Eine therapeutisch wirksame Menge an ITPP zur Verwendung nach Anspruch 1, wobei das Chemotherapeutikum ein Inhibitor des Nukleosidmetabolismus ist, der aus Gemcitabin und Capecitabin ausgewählt ist.

11. Eine therapeutisch wirksame Menge an ITPP zur Verwendung nach Anspruch 1, wobei ITPP etwa 2 Stunden bis 5 Tage vor der Verabreichung des Chemotherapeutikums verabreicht wird.

12. Eine therapeutisch wirksame Menge an ITPP gemäß der Verwendung nach Anspruch 1, wobei der Krebs ausgewählt ist aus soliden Tumoren, umfassend Sarkome, Karzinome, Keimbahn-Tumoren, Tumoren des zentralen Nervensystems, Brustkrebs, Prostatakrebs, Gebärmutterhalskrebs, Gebärmutterkrebs, Lungenkrebs, Eierstockkrebs, Hodenkrebs, Schilddrüsenkrebs, Astrozytom, Gliom, Bauchspeicheldrüsenkrebs, Magenkrebs, Leberkrebs, Darmkrebs, Melanom. Nierenkrebs, Blasenkrebs, Krebs der Speiseröhre, Krebs des Kehlkopfes, Krebs des Parotis, Krebs des Gallenganges, Rektumkarzinom, Endometriumkrebs, Plattenepithelkarzinome, Adenokarzinome, kleinzellige Karzinome, Neuroblastome, Mesotheliome, adrenokortikale Karzinome, Epithelkarzinome, desmoide Tumoren, desmoplastische kleine runde Zelltumoren, endokrine Tumore, Ewing-Sarkom-Familientumoren, Keimzelltumoren, Hepatoblastome, hepatozelluläre Karzinome, Lymphome, Melanome, Non-Rhabdomyo-Weichteilsarkomen, Osteosarkome, periphere primitive neuroektodermale Tumore, Retinoblastome, Rhabdomyosarkome und Wilms-Tumoren.

13. Eine therapeutisch wirksame Menge an ITPP zur Verwendung nach Anspruch 12, wobei der Krebs Melanom ist.

14. Eine therapeutisch wirksame Menge an ITPP zur Verwendung nach Anspruch 12, wobei der Krebs Bauchspeicheldrüsenkrebs ist.

15. Ein Kit zur Behandlung von soliden Tumoren und zur Verringerung der Metastasierung, umfassend Inositoltrispyrophosphat (ITPP) und ein Chemotherapeutikum, wobei das Chemotherapeutikum ein Mikrotubuli-Targeting-Agens, ein DNA-Interkalations-Agens oder ein Inhibitor des Nukleosidmetabolismus ist.

16. Ein Kit nach Anspruch 15, wobei das Chemotherapeutikum ein Mikrotubuli-Targeting-Agens ist, das aus Docetaxel und Paclitaxel ausgewählt ist.

17. Ein Kit nach Anspruch 15, wobei das Chemotherapeutikum das DNA-Interkalations-Agens ist, das aus Platin-basierten Agenzien und Doxorubicin ausgewählt ist.

18. Ein Kit nach Anspruch 17, wobei das Platin-basierte Agens Cisplatin ist.

19. Ein Kit nach Anspruch 18, wobei das Cisplatin mit Paclitaxel kombiniert ist.

20. Ein Kit nach Anspruch 15, wobei das Chemotherapeutikum ein Inhibitor des Nukleosidmetabolismus ist, der aus Gemcitabin und Capecitabin ausgewählt ist.

21. Ein Kit nach Anspruch 15, wobei das Kit ferner die Anweisungen zur Verabreichung einer therapeutisch wirksamen Menge an Inositoltrispyrophosphat (ITPP) bei einem Krebspatienten vor der Verabreichung einer wirksamen Menge des Chemotherapeutikums bereitstellt.

22. Eine therapeutisch wirksame Menge an ITPP zur Verwendung nach Anspruch 1, wobei ITPP die Metastasierung hemmt.

## Revendications

1. Quantité thérapeutiquement efficace d'inositol trispyrophosphate (ITPP) pour une utilisation dans une normalisation vasculaire partielle d'une tumeur chez un patient atteint d'un cancer avant l'administration d'une quantité efficace d'un agent chimiothérapeutique, dans laquelle l'agent chimiothérapeutique est :
un agent de ciblage de microtubules choisi parmi de la colchicine, du docétaxel, du paclitaxel, de la vinblastine, de la vincristine, de la vindésine, et de la vinorelbine ;
un agent d'intercalation d'ADN choisi parmi du cisplatine, de la daunorubicine, de la doxorubicine, ou de l'épirubicine ; ou
un inhibiteur métabolique de nucléosides choisi parmi de la cytarabine, de la fludarabine, de la fluorouracile, de la gemcitabine, de la mercaptopurine, ou de la pentostatine.

2. Quantité thérapeutiquement efficace d'ITPP pour une utilisation selon la revendication 1, comprenant en outre une détection de l'occurrence d'une normalisation vasculaire partielle de la tumeur.

3. Quantité thérapeutiquement efficace d'ITPP pour une utilisation selon la revendication 2, dans laquelle l'occurrence d'une normalisation vasculaire partielle est détectée par mesure d'un niveau de pression d'oxygène partielle (pO2) de la tumeur.

4. Quantité thérapeutiquement efficace d'ITPP pour une utilisation selon la revendication 1, dans laquelle l'agent chimiothérapeutique est administré dans une dose sous-thérapeutique.

5. Quantité thérapeutiquement efficace d'ITPP pour une utilisation selon la revendication 1, dans laquelle le cancer est résistant à un ou plusieurs agents chimiothérapeutiques et une efficacité des agents chimiothérapeutiques contre des cellules tumorales est augmentée.

6. Quantité thérapeutiquement efficace d'ITPP pour une utilisation selon la revendication 1, dans laquelle l'agent chimiothérapeutique est un agent de ciblage de microtubules qui est choisi parmi du docétaxel et du paclitaxel.

7. Quantité thérapeutiquement efficace d'ITPP pour une utilisation selon la revendication 1, dans laquelle l'agent chimiothérapeutique est un agent d'intercalation d'ADN qui est choisi parmi des agents à base de platine et de la doxorubicine.

8. Quantité thérapeutiquement efficace d'ITPP pour une utilisation selon la revendication 7, dans laquelle l'agent à base de platine est du cisplatine.

9. Quantité thérapeutiquement efficace d'ITPP pour une utilisation selon la revendication 5, dans laquelle le cisplatine est combiné à du paclitaxel dans le traitement d'un mélanome et d'une métastase pulmonaire.

10. Quantité thérapeutiquement efficace d'ITPP pour une utilisation selon la revendication 1, dans laquelle l'agent chimiothérapeutique est un inhibiteur métabolique de nucléosides qui est choisi parmi de la gemcitabine et de la capécitabine.

11. Quantité thérapeutiquement efficace d'ITPP pour une utilisation selon la revendication 1, dans laquelle de l'ITPP est administré environ 2 heures à 5 jours avant l'administration de l'agent chimiothérapeutique.

12. Quantité thérapeutiquement efficace d'ITPP pour une utilisation selon la revendication 1, dans laquelle le cancer est choisi parmi des tumeurs solides incluant des sarcomes, des carcinomes, des tumeurs de lignée germinale, des tumeurs du système nerveux central, un cancer du sein, un cancer de la prostate, un cancer cervical, un cancer de l'utérus, un cancer du poumon, un cancer de l'ovaire, un cancer du testicule, un cancer de la thyroïde, un astrocytome, un gliome, un cancer du pancréas, un cancer de l'estomac, un cancer du foie, un cancer du côlon, un mélanome, un cancer du rein, un cancer de la vessie, un cancer de l'oesophage, un cancer du larynx, un cancer de la parotide, un cancer du tractus biliaire, un cancer du rectum, un cancer de l'endomètre, des carcinomes des cellules squameuses, des adénocarcinomes, des carcinomes à petites cellules, des neuroblastomes, des mésothéliomes, des carcinomes corticosurrénaliens, des carcinomes épithéliaux, des tumeurs desmoïdes, des tumeurs desmoplastiques de petites cellules rondes, des tumeurs endocrines, des tumeurs du type sarcome d'Ewing, des tumeurs de cellules germinales, des hépatoblastomes, des carcinomes hépatocellulaires, des lymphomes, des mélanomes, des sarcomes des tissus mous non rhabdomyosarcomes, des ostéosarcomes, des tumeurs neuroectodermiques primitives périphériques, des rétinoblastomes, des rhabdomyosarcomes, et des tumeurs de Wilms.

13. Quantité thérapeutiquement efficace d'ITPP pour une utilisation selon la revendication 12, dans laquelle le cancer est un mélanome.

14. Quantité thérapeutiquement efficace d'ITPP pour une utilisation selon la revendication 12, dans laquelle le cancer est un cancer du pancréas.

15. Kit destiné au traitement de tumeurs solides et à la réduction de métastases comprenant de l'inositol trispyrophosphate (ITPP) et un agent chimiothérapeutique, dans lequel l'agent chimiothérapeutique est un agent de ciblage de microtubules, un agent d'intercalation d'ADN, ou un inhibiteur métabolique de nucléosides.

16. Kit selon la revendication 15, dans lequel l'agent chimiothérapeutique est un agent de ciblage de microtubules qui est choisi parmi du docétaxel et du paclitaxel.

17. Kit selon la revendication 15, dans lequel l'agent chimiothérapeutique est l'agent d'intercalation d'ADN qui est choisi parmi des agents à base de platine et de la doxorubicine.

18. Kit selon la revendication 17, dans lequel l'agent à base de platine est du cisplatine.

19. Kit selon la revendication 18, dans lequel le cisplatine est combiné à du paclitaxel.

20. Kit selon la revendication 15, dans lequel l'agent chimiothérapeutique est un inhibiteur métabolique de nucléosides qui est choisi parmi de la gemcitabine et de la capécitabine.

21. Kit selon la revendication 15, dans lequel le kit comprend en outre les instructions pour une administration d'une quantité thérapeutiquement efficace d'inositol trispyrophosphate (ITPP) chez un patient atteint d'un cancer avant l'administration d'une quantité efficace dudit agent chimiothérapeutique.

22. Quantité thérapeutiquement efficace d'ITPP pour une utilisation selon la revendication 1, dans laquelle l'ITPP inhibe des métastases.
